# EUROPEAN PATENT APPLICATION

(11) **EP 1 285 698 A1**
(43) Date of publication of application: **26.02.2003**
(21) Application number: 01948171.2
(22) Date of filing: 21.06.2001
(51) Int. Cl.: B03C 3/00, A61L 9/22

(54) **DEVICE FOR CLEANING AIR FROM DUST AND AEROSOLS**

(30) Priority: 27.03.2001 RU 2001107867
(71) Applicant: Obschestvo S Ogranichennoi Otvetstvennostju "Obnovlenie", Orel, 302028 (RU)
(72) Inventor: KOTLYAR, Gennady Mikhailovich, Orel, 302028 (RU); SYSOEV, Ivan Vasilievich, Orel, 302028 (RU)
(74) Representative: Jehan, Robert
(86) International application number: PCT/RU01/00247
(87) International publication number: WO 02/076619

(57) **Abstract**

A device for air cleaning from dust and aerosols based on the use of non-homogeneous electrostatic field creating a flow of charged particles (the so called "ionic wind") and operating as electrostatic precipitator is proposed. The device comprises a bode 1 inside which corona-forming (3) and precipitation (4) electrodes with opposite polarities are established. Inside the body (1) deflection electrodes (%) are established as well, in front of corona-forming (3) electrodes a reflector for positively charged aeroions effected as electrode (6) and electrically coupled with said corona-forming (3) electrodes being established. The proposed device comprises one or several tapes made of porous-fiber material placed into non-homogeneous electric field.

## Description

### Technical Field

The present invention relies to separation of disperse particles with the use of the electrostatic effect, to be more precise, the present invention relies to devices aimed at air cleaning from dust and aerosols. The invention can find application in all fields of industry as well as in domestic rooms.

### Background art

A device comprises a body with an air inlet, corona-forming and precipitation electrodes of opposite polarities. Near precipitation electrodes, an electrostatic precipitation element is installed. Said element is effected of two metal nets with precipitating fabric set in-between (for ref. see Inventor's Certificate of the USSR No. 921629, class B 03 C 3/08).

The above-mentioned known device does not avoid extraction of harmful for health positively charged aeroions as well as it does not provide for saturation of cleaned air with molecules of aromatic or medical substances if necessary.

As to its principle of operation the proposed device is closest to a device described in the Patent of Russian Federation No. 2159683, class B C 3/04 published in 2000. This known device comprises a body with an air inlet, inside the body corona-forming and precipitation electrodes with opposite polarities, passive electrodes are set, behind electrodes an electrode - generator of negative electrodes being electrically coupled with precipitation electrodes and a vessel with aroma or medical substances are mounted.

The principle of work of the prior art device is based on the forming of charged particles in the field of corona-forming charge appearing between positively charged corona and negatively charged precipitation electrodes. Corona-forming and deflecting electrodes have similar polarities but different electrical potential in relation to precipitation electrodes. The presence of deflection electrodes increases level of purification of air greatly (up to 95 - 99 per cent). Passive electrode acquiring positive charge prevents positively charged aeroions from leaving air cleaner thus preventing an accommodation from being polluted with harmful positively charged aeroions. Said air cleaner while operating provides forming of negatively charged aeroions of oxygen in great amounts. A part of these ions being seized by means of dust or aerosols particles are fallen on passive electrodes thus covering electrodes with dielectric fine dispersion layer. This layer of dust partially neutralizes positively charged electrodes but mainly it prevents from its appearance thus decreasing efficiency of the passive electrode as a precipitator of positively charged ones greatly which leads to the necessity of producing quite often cleaning of the passive electrodes thus effecting some inconveniences while exploitation.

The presence of a vessel allows performing saturation of air with aroma substances, however, liquid overflowing out of the vessel is mixed with dust fallen, makes inner surfaces dirty, worsens electric isolation safety and may produce electrical shorting. Besides, the intensity of liquid evaporation in a not- homogeneous electric field is rather great, small amounts of liquid are evaporated very fast thus requiring often recharges of the vessel with aroma or medical liquids. As the vessel is mounted inside air cleaner, such recharges are connected, as a rule, with partial or full reassembling of the device which leads to additional time and facilities expenditures. High aerodynamic resistance reduces efficiency of the device.

### Disclosure of the invention

The invention is based on the problem of providing a device for purifying of air from dust and aerosols, which, along with providing a possibility to saturate air with aroma and medical substances is convenient at its exploitation and has a higher possibility of complex air treatment.

These objects are accomplished by that in a device for air purification from dust and aerosols comprising a body with a air inlet, inside said body corona and collecting electrodes are mounted having opposite polarities, deflecting electrodes behind which an electrode-generator of negatively charged electrons is mounted. Said generator is electrically coupled with precipitation electrodes. In the body a means for aroma and medical substances is mounted according to the invention in front of corona-forming electrodes a reflector of positively charged electrons is set being electrically coupled with corona-forming electrodes, while a means for aroma or medical substances is effected as one or few tapes made of porously-fiber material which ends are included into a vessel with aroma or medical substances being mounted beyond the body.

The proposed device allows avoiding exclusions of harmful for health positively charged aeroions in conditions of any long terms operation, but an additional cleaning of a reflector of aeroions from dust is not required. Direct measurements has shown that concentration of positively charged aeroions at inlet is decreased to tens of thousand, as well as background values of positively charged aeroions concentration at inlet of the device. This can be explained by the fact that positively charged reflector pushes away aeroions that are at the inlet of air cleaner. While operation, dielectric fine dispersion dust is collected on reflector, nevertheless this fact does not lessen its efficiency, as it is constantly remains under high positive potential. A layer of dielectric fine dispersion dust does not influence much on dispersion of power lines of electrostatic field of a reflector. Thus, cleaning of the reflector is not necessary and it can be combined with cleaning of precipitation and deflecting electrodes if necessary.

Replacement of a vessel with a tape made of porously-fiber material (as a wick), which ends are placed into capacities with aroma or medical substances, allows reducing aerodynamic resistance to air flow greatly as thickness of the tape is not more than several millimeters. At the same time a possibility of water to drop on precipitation and deflection electrodes is avoided as well as free (open) surface of liquid is increased. As the capacities for liquids are placed beyond operation zone of the device, refilling of the capacities can be performed without switching the device off the power source through special aperture by means of syringe or other known method. There are no any limits in size of capacities for aroma or medical substances in the proposed device. Instead of one tape made of porously-fiber material in the proposed device it is possible to use two or even more tapes which ends are placed into capacities with aroma or medical substances, thus allowing forming bouquets of fragrances or medical mixtures.

To use the device in ozonization rate, according to the invention, accelerating electrode, to which a controlled positive potential is provided, is placed in some distance from electrode-generator, said accelerating electrode is effected with a possibility of its movement in relation to electrode-generator.

According to the invention, high-frequency alternating current is provided to accelerating electrode.

To increase productivity and to raise coefficient of purification of air from dust and aerosols in the device, according to the invention, a few reflectors of positively charged aeroions, corona-forming, precipitation and deflection electrodes are placed in sequence.

### Brief description of drawings

The present invention will now be described in greater detail with reference to various specific embodiments thereof taken in conjunction with the accompanying drawings, in which:
Fig. 1 illustrates a general embodiment of the proposed device;
Fig. 2 illustrates one of the embodiments of the proposed device.

### Detailed Description of the Preferred Embodiments

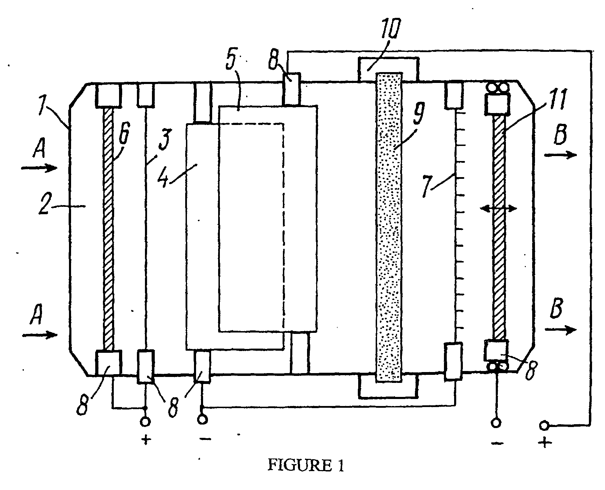

The proposed device comprises a body 1 (Fig.1) with valve 2 for air, said valve having inlet and outlet (not shown in Fig.1), air input to canal 2 in the drawing being shown by means of "A" arrow, while air output is shown by means of "B" arrow. Inside the body corona-forming electrodes 3 (positively charged) and precipitation electrodes 4 (negatively charged), as well as deflecting electrodes 5 positively charged are established. Voltage volume supplied to electrodes 5 is less than voltage volume supplied to electrodes 3 and 4. In front of corona-forming electrodes 3 and parallel to each of them a reflector of positively charged aeroions is established at a distance. Said reflector is effected as electrodes 6 electrically coupled with electrodes 3, electrodes 6 having bigger diameter than that of electrodes 3.

Behind deflection electrodes 5 an electrode-generator 7 of negatively charged aeroions is established, being electrically coupled with precipitation electrodes 4. Said electrode-generator 7 may be effected as electro-conducting net made of thin wire and is equipped with concentrators effected as needles.

Corona-forming electrodes 3 in this particular embodiment are effected as thin electricity conducting threads made of tungsten wire of a relative diameter while reflector-electrodes 6 are effected as rods made of stainless steel of round-section with diameter 10 - 20 times as big as that of electrodes 3. Precipitation 4 and deflection 5 electrodes are effected as electricity conducting plates. All above mentioned electrodes are established in body 1 made of dielectric on electricity isolators 8.

In front of electrode 7 one or several tapes 9 made of porously-fiber material (wick) which ends are placed in vessels 10 with aromatic or medical substances, placed beyond the body 1. Tapes 9 are placed in the zone of the most-homogeneous electric field - i.e., between deflection electrodes 5 and electrode - generator 7 of negatively charged aeroions.

To use the device in ozonization rate, behind electrode-generator 7 at a distance an accelerating electrode 11 is established, a controlled positive potential is supplied to said electrode 11, between electrode-generator 7 and accelerating electrode 11 a controlled non-homogeneous electric field is formed. Change in volume and gradient of said field is effected by means of either change of voltage volume between electrodes 7 and 11, o change of a distance between said electrodes due to replacement of electrode 11 in respect to electrode-generator 7.

To increase the productivity of ozone, change of corona-forming 3, precipitation 4 and deflection 5 electrodes' polarities is provided. Instead of direct current a high frequency alternative current (50 kHz and up) may be supplied to electrodes 7 and 11 thus increasing volume of ozone produced.

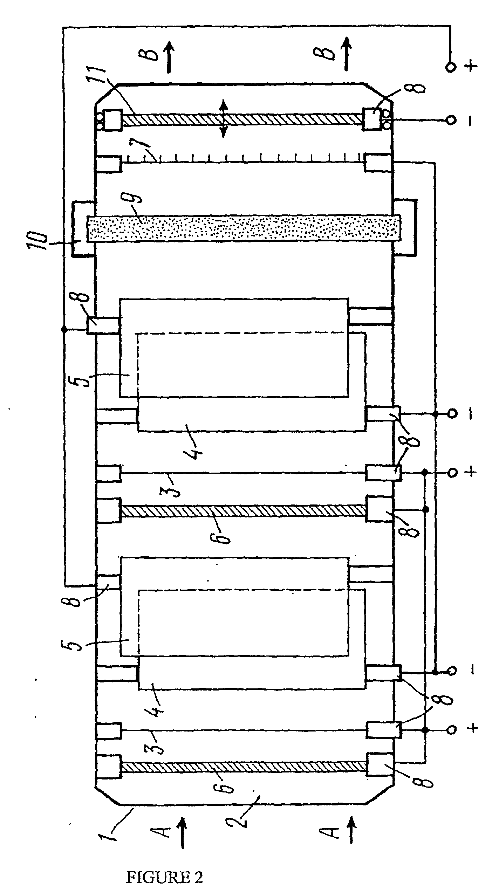

To enhance productivity and a coefficient of cleaning air from dust and aerosols one of the embodiments of the device is presented in Fig.2

Under such variant of the embodiment several reflectors 6 of positively charged aeroions, several corona0forminf 3, precipitation 4 and deflection 5 electrodes are used.

The proposed device operates as follows:

Under supply of high voltage to corona-forming 3 and precipitation 4 electrodes a corona discharge appears, thus forming a flow of positively charged nitrogen and oxygen ions directed towards precipitation electrodes 4, this effect is called "ionic wind" Together with aeroions in the non-homogeneous electric field neutral molecules as well as particles of dust and aerosols contained in the air and moving towards precipitation electrodes are being polarizes and charged. Particles of dust and aerosol after being positively charged are dropped to precipitation electrodes 4 while negatively charged particles are dropped to deflection electrodes 5, besides said particles produce slowing-down effect to very fine positively charged particles which due to their high velocity can not be dropped to precipitation electrodes. Due to deflection electrodes 5 level of purification reaches 95 - 99 per cent. Electric field of reflector 6 having positive potential prevents harmful positively charged aeroions from leaving the device, it changes the direction of their movement to the opposite one thus directing particles towards precipitation electrodes 4 thus increasing air purity. Corona-forming electrodes 7 established behind deflection electrodes 5 saturate the air with negatively charged aeroions of oxygen. A part of positively charged aeroions having passed through slowing-down field of deflection electrode 5 is being changed when entering a zone filled with negatively charged aeroions of oxygen and electrons. In this case concentration of positively charged aeroions at outlet is reduced considerately to their background level while concentration of useful negatively charged aeroions increases greatly. Direct measurements prove that in this particular case ozone is almost absent.

During ozonization rate negative potential is supplied to corona-forming electrodes 3, positive potential is supplied to precipitation electrodes 4, while deflection electrodes 5 are supplied with negative potential of less value. To increase the amount of ozone produced electrodes 7 and 11 are supplied with high voltage, which volume can be changed, thus, allowing controlling of ozone production in wide range. It is noticeable that the efficiency of air cleaning from dust and aerosols under this particular rate is not lower than that in conditions of positive corona-forming while the amount of negatively charged oxygen aeroions is much greater.

Aromatic or medical substances are transferred through tape 9 made of porously-fiber material from vessels 10 to the zone of non-homogeneous electricity field under forces of inter-molecular interaction (known as capillary phenomenon). In said non-homogeneous field additionally Koulon forces are applied to polarized molecules of liquid, said forces are directed towards the raise in tension of electrical field. These forces reduce attraction between molecules thus promoting faster evaporation of aromatic or medical substances. Control over the intense of evaporation of liquid is provided by the fact that open (free) surface of liquid in the tape of porously-fiber material may be controlled in wide range. By means of cleaned air flow molecules of aromatic or medical substances together with negatively charged aeroions of oxygen are taken beyond air cleaner. Both the method described and the device proposed do not require any dissolvent polluting environment, as well as special sprays, inhalators and fans. While ozonization rate air saturation with aromatic or medical substances is not recommended as non-controlled oxidation of said substances by means of ozone may take place.

### Industrial applicability

The proposed device can find its application both in all kinds of industry and in domestic rooms for saturation of air with molecules of aromatic and medical substances, negatively charged oxygen aeroions as well as with controlled amount of ozone.

## Claims

1. A device for air cleaning from dust and aerosols comprising a body (1) with an air inlet (2), inside the body corona-forming (3) and precipitation (4) electrodes (5) with opposite polarities, deflection electrodes are established. Behind said deflection electrodes electrode-generator (7) of negatively charged aeroions is established being electrically coupled with precipitation electrodes (4) as well as a means for aromatic or medical substances **CHARACTERIZED IN that** in front of corona-forming electrodes (3) a reflector (6) of positively charged aeroions is established being electrically coupled with said corona-forming electrodes (3), while a means for aromatic or medical substances is effected as one or several tapes (9) made of porously-fiber material, which ends are placed in capacities (10) containing aromatic or medical substances, said capacities are places beyond the body (1) of the proposed device.

2. A device according to claim 1 **CHARACTERIZED IN that** behind electrode-generator (7) at a distance an accelerating electrode (11) is established, to which a controlled positive potential is supplied.

3. A device according to claim 2 CHARACERIZED IN that electrode (11) is effected with a possibility of movement in respect to electrode-generator (7);

4. A device according to claim 2 **CHARACTERIZED IN that** a high-frequency alternating current is supplied to electrode-generator (7).

5. A device according to any of claims 1 - 4 **CHARACTERIZED IN that** it comprises several reflectors (6) of positively charged aeroions, corona-forming (3), precipitation (4) and deflection (5) electrodes places in sequence.
